# EUROPEAN PATENT APPLICATION

(11) **EP 4 502 615 A1**
(43) Date of publication of application: **05.02.2025**
(21) Application number: 23780782.1
(22) Date of filing: 29.03.2023
(51) Int. Cl.: G01N 35/00

(54) **AUTOMATIC ANALYSIS DEVICE**

(30) Priority: 30.03.2022 JP 2022056531
(71) Applicant: Sekisui Medical Co., Ltd., Tokyo 103-0027 (JP); Canon Medical Systems Corporation, Otawara-shi, Tochigi 324-8550 (JP)
(72) Inventor: KAWABE, Toshiki, Tokyo 103-0027 (JP); OGASAWARA, Kosuke, Tokyo 103-0027 (JP); IIJIMA, Yumi, Tokyo 103-0027 (JP); YAMASAKI, Kenji, Ohtawara-shi, Tochigi 324-8550 (JP); SHINOHARA, Hiroo, Ohtawara-shi, Tochigi 324-8550 (JP)
(74) Representative: Maiwald GmbH
(86) International application number: PCT/JP2023/013001
(87) International publication number: WO 2023/190774

(57) **Abstract**

Obtained is an automated analysis device capable of efficiently executing coagulation measurement and measurement different from the coagulation measurement.

Provided are: a recording unit that records one or more measurement items performed on a specimen put in at least one specimen container and a measurement order of each of the measurement items; a specimen dispensing mechanism 50 that dispenses the specimen selected as a measurement target based on the measurement order into at least one of a first reaction container or a second reaction container; a first measurement unit 25 that performs coagulation measurement, which is measurement of a coagulation item, on the specimen dispensed into the first reaction container after completion of a first preparation process determined in advance; and a second measurement unit 35 that performs specific measurement, which is the measurement item different from the measurement of the coagulation item, on the specimen dispensed into the second reaction container after completion of a second preparation process determined in advance. In response to the completion of the first preparation process, the first measurement unit can execute the measurement of the coagulation item regardless of whether the second preparation process is completed.

## Description

### Technical Field

The present disclosure relates to an automated analysis device capable of analyzing a coagulation item and items other than the coagulation item and capable of quickly obtaining a result of measurement of the coagulation item (hereinafter, also referred to as coagulation measurement).

### Background Art

Japanese Patent Application Laid-Open (JP-A) No. 2001-13151 discloses an automated analysis device that analyzes blood of a subject.

### SUMMARY OF INVENTION

### Technical Problem

By the way, in the coagulation measurement, since it is required to acquire a measurement result more quickly, it is desirable for automated analysis devices to efficiently execute the coagulation measurement and measurement (which is measurement of an item different from the coagulation item and hereinafter also referred to as specific measurement) different from the coagulation measurement. There is room for improvement in the automated analysis device of JP-A No. 2001-13151 in terms of efficiently executing coagulation measurement for blood (specimen) of a subject and measurement different from the coagulation measurement.

In consideration of the above fact, an object of the disclosure is to obtain an automated analysis device capable of efficiently executing coagulation measurement and measurement different from the coagulation measurement.

### Solution to Problem

An automated analysis device according to a first aspect of the disclosure includes: a recording unit that records one or more measurement items performed on a specimen placed in at least one specimen container and records a measurement order of each of the measurement items; a specimen dispensing mechanism that dispenses the specimen selected as a measurement target based on the measurement order into at least one of a first reaction container or a second reaction container; a first measurement unit that performs coagulation measurement, which is measurement of a coagulation item, on the specimen dispensed into the first reaction container after completion of a first preparation process determined in advance; and a second measurement unit that performs specific measurement, which is a measurement item that is different from the measurement of the coagulation item, on the specimen dispensed into the second reaction container after completion of a second preparation process determined in advance. In response to the completion of the first preparation process, the first measurement unit can execute the measurement of the coagulation item regardless of whether the second preparation process is completed.

An automated analysis device according to a second aspect of the disclosure is the automated analysis device of the first aspect, in which a time required for the second preparation process is longer than a time required for the first preparation process.

An automated analysis device according to a third aspect of the disclosure is the automated analysis device according to the first aspect or the second aspect, in which the first measurement unit and the second measurement unit are separated from each other.

An automated analysis device according to a fourth aspect of the disclosure is the automated analysis device according to the third aspect, in which the first measurement unit includes: a first support unit that supports a plurality of first reaction containers into which the specimen dispensed by the specimen dispensing mechanism is placed; a first measurement execution unit that performs the coagulation measurement on the specimen in the first reaction containers; and a first movement unit that moves the first reaction containers on which the coagulation measurement has been executed from the first support unit to an outside of the first support unit without cleaning, the second measurement unit includes: a second support unit that supports a plurality of the second reaction containers into which the specimen dispensed by the specimen dispensing mechanism is placed; a second measurement execution unit that performs the specific measurement on the specimen in the second reaction containers; and a cleaning unit that cleans the second reaction containers on which the specific measurement has been executed, and the second preparation process includes a cleaning process which is performed on all of the second reaction containers by the cleaning unit.

An automated analysis device according to a fifth aspect of the disclosure is the automated analysis device according to any one of the first to fourth aspects, in which the first measurement unit performs the coagulation measurement regardless of whether the second preparation process is completed, in response to the completion of the first preparation process in a case in which the measurement item performed on the specimen in the specimen container is only the coagulation measurement or includes the coagulation measurement and only the specific measurement executed after the coagulation measurement.

An automated analysis device according to a sixth aspect of the disclosure is the automated analysis device according to any one of the first to fifth aspects, in which the first preparation process includes a process of stirring a reagent used for the coagulation measurement.

An automated analysis device according to a seventh aspect of the disclosure is the automated analysis device according to any one of the first to sixth aspects, further including: a first support unit that is a part of the first measurement unit and supports a plurality of first reaction containers into which the specimen dispensed by the specimen dispensing mechanism is placed; a container supply unit that stores the plurality of first reaction containers; and a conveyance mechanism that is capable of conveying the first reaction containers accommodated in the container supply unit to the first support unit and conveying the first reaction containers from the first support unit to a predetermined position. The first preparation process includes a process of conveying the first reaction containers to the first support unit using the conveyance mechanism and a process of conveying the first reaction container from the first support unit using the conveyance mechanism.

An automated analysis device according to an eighth aspect of the disclosure is the automated analysis device according to any one of first to seventh aspects, further including: a first support unit that is a part of the first measurement unit and supports a plurality of the first reaction containers into which the specimen dispensed by the specimen dispensing mechanism is placed; and a first temperature adjustment unit that performs thermostatic control of a temperature of the first support unit at a constant temperature. The first preparation process includes a thermostatic control process of the first support unit by the first temperature adjustment unit.

An automated analysis device according to a ninth aspect of the disclosure is the automated analysis device according to any one of the first to eighth aspects, further including: a second support unit that is a part of the second measurement unit and supports a plurality of the second reaction containers into which the specimen dispensed by the specimen dispensing mechanism is placed; and a cleaning unit that cleans the second reaction container supported by the second support unit. The second preparation process includes a process of cleaning all the second reaction containers supported by the second support unit, the process being executed by the cleaning unit.

An automated analysis device according to a tenth aspect of the disclosure is the automated analysis device according to any one of the first to ninth aspects, further including: a second support unit that is a part of the second measurement unit and supports a plurality of the second reaction containers into which the specimen dispensed by the specimen dispensing mechanism is placed; and a system water supply unit that supplies system water to the second reaction containers. The second preparation process includes a system water measurement process by the second measurement unit.

An automated analysis device according to an eleventh aspect of the disclosure is the automated analysis device according to any one of the first to tenth aspects, further including: a second support unit that is a part of the second measurement unit and supports a plurality of second reaction containers into which the specimen dispensed by the specimen dispensing mechanism is placed; and a contamination detection unit that detects a contamination state of the second reaction containers. The second preparation process includes a contamination detection process of the second reaction container by the contamination detection unit.

An automated analysis device according to a twelfth aspect of the disclosure is the automated analysis device according to any one of the first to eleventh aspects, further including: a second support unit that is a part of the second measurement unit and supports a plurality of second reaction containers into which the specimen dispensed by the specimen dispensing mechanism is placed; and a second temperature adjustment unit that performs thermostatic control of a temperature of the second support unit. The second preparation process includes a thermostatic control process of the second support unit by the second temperature adjustment unit.

### Advantageous Effects of Invention

With the automated analysis device according to the disclosure, the coagulation measurement of the blood specimen and the measurement different from the coagulation measurement can be efficiently executed.

### BRIEF DESCRIPTION OF DRAWINGS

Fig. 1 is a perspective view of an automated analysis device according to an embodiment of the disclosure as viewed from the front.
Fig. 2 is a schematic cross-sectional view of the automated analysis device taken along line 2-2 of Fig. 1.
Fig. 3 is a control block diagram of a control unit of the automated analysis device.
Fig. 4 is a functional block diagram of the control unit.
Fig. 5 is a perspective view of a specimen rack and a specimen container set in the automated analysis device.
Fig. 6 is a view showing a state transition of the analysis device.
Fig. 7 is a flowchart showing processing executed by the control unit.
Fig. 8 is a flowchart showing processing executed by the control unit.
Fig. 9 is a schematic plan view of the specimen rack and the specimen container.
Fig. 10 is a view showing types of measurement contents of a specimen container of k = 1.
Fig. 11 is a view showing types of measurement contents of a specimen container of k = 2.
Fig. 12 is a view showing a combination of measurement items in the case of N = 2.
Fig. 13 is a flowchart showing processing executed by a control unit according to a modification.
Fig. 14 is a view showing times required for coagulation measurement and specific measurement preparation processes according to another modification.
Fig. 15 is a view showing times required for coagulation measurement and specific measurement preparation processes according to still another modification.

### DESCRIPTION OF EMBODIMENTS

Hereinafter, an automated analysis device 10 (hereinafter, the analysis device 10) according to an embodiment of the disclosure will be described with reference to the accompanying drawings. Note that an arrow FR appropriately shown in each drawing indicates a forward direction, an arrow UP indicates an upward direction, and an arrow LH indicates the left in the left-right direction. In the drawings, the same or corresponding parts may not repeatedly denoted by the same reference numerals.

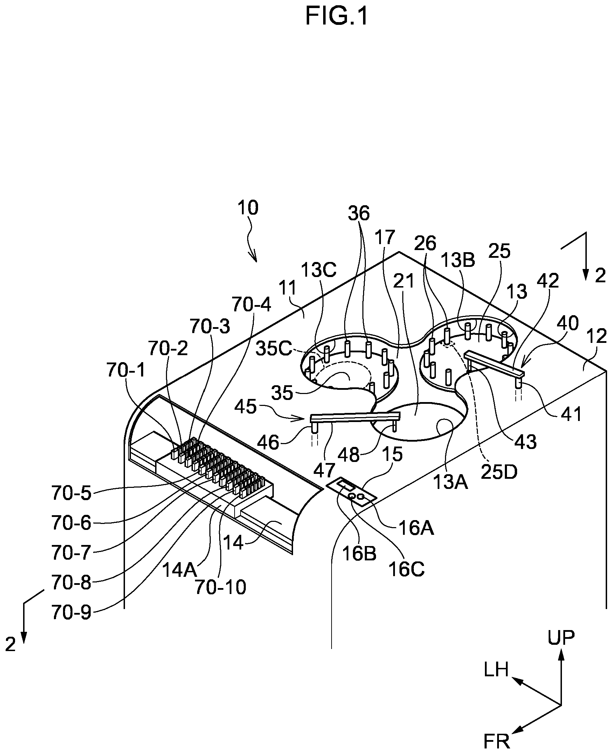

The analysis device 10 shown in Fig. 1 can perform coagulation measurement and specific measurement (for example, colorimetric measurement) on a specimen that is blood (plasma) collected from a subject. Note that the coagulation measurement in the present specification is measurement for obtaining a coagulation time as a measurement result or a concentration or an activity value derived based on the coagulation time. The analysis device 10 has a housing 11 having a substantially rectangular parallelepiped shape. A top of the housing 11 is formed of a horizontal top plate 12. An opening 13 is formed in the top plate 12. The opening 13 includes a first opening 13A, a second opening 13B, and a third opening 13C each having a substantially circular shape. Further, a rack accommodating portion 14, which is a recessed portion extending in the left-right direction, is formed at a front end portion of a top of the housing 11. The front end portion of the housing 11 is provided with a transparent cover member (not shown)-that can open and close the rack accommodating portion 14.

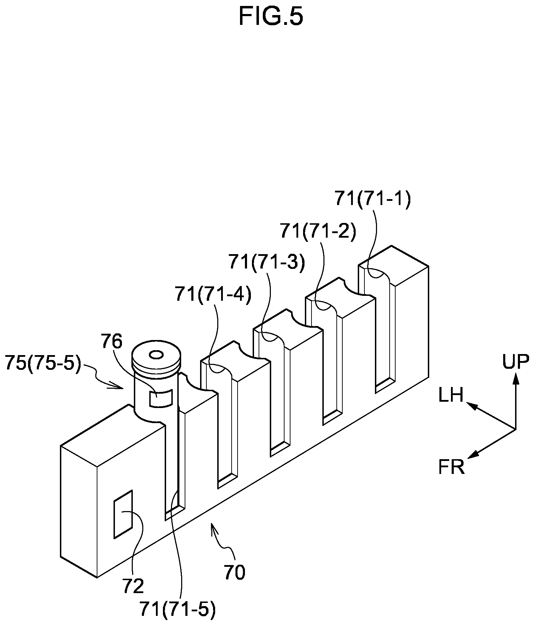

As shown in Fig. 1, a rack tray 14A is detachably provided at the bottom of rack accommodating portion 14. In the rack tray 14A, specimen rack 70 shown in Fig. 5 can be placed. In the rack tray 14A of the present embodiment, ten specimen racks 70 can be placed. As shown in Fig. 1, the ten specimen racks 70 here are referred to as specimen racks 70-1, 70-2, 70-3, 70-4, 70-5, 70-6, 70-7, 70-8, 70-9, and 70-10 in order from the left. As shown in Fig. 5, the specimen rack 70 includes five support grooves 71. A specimen container 75, which is a vertically long container such as a blood collection tube, is detachably provided in each of the support grooves 71. In each of the specimen containers 75, blood of the subject and a sodium citrate solution as an anticoagulant are accommodated so as to have a predetermined capacity ratio. The specimen container 75 has been centrifuged by a centrifuge (not shown) such that the specimen container accommodates plasma at the upper side and blood cells at the lower side.

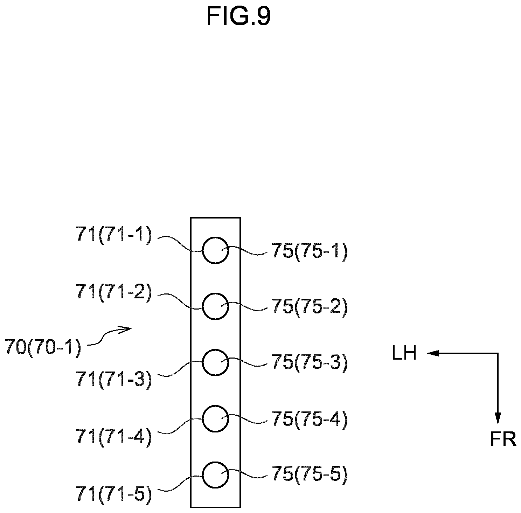

In a case in which the longitudinal direction of the specimen rack 70 is parallel to the front-rear direction as shown in Fig. 5, the five support grooves 71 may be also referred to as support grooves 71-1, 71-2, 71-3, 71-4, and 71-5 in order from the rear. As shown in Figs. 5 and 9, the specimen containers 75 provided in the support grooves 71-1, 71-2, 71-3, 71-4, and 71-5 may be also referred to as specimen containers 75-1, 75-2, 75-3, 75-4, and 75-5, respectively.

As shown in Fig. 5, each of the specimen racks 70 is provided with a rack barcode 72 (for example, a two-dimensional code) on which ID information for identifying each of the specimen racks 70 is printed. In addition, a specimen barcode 76 on which ID information for identifying a specimen contained in each of the specimen containers 75 is printed is pasted to each of the specimen containers 75.

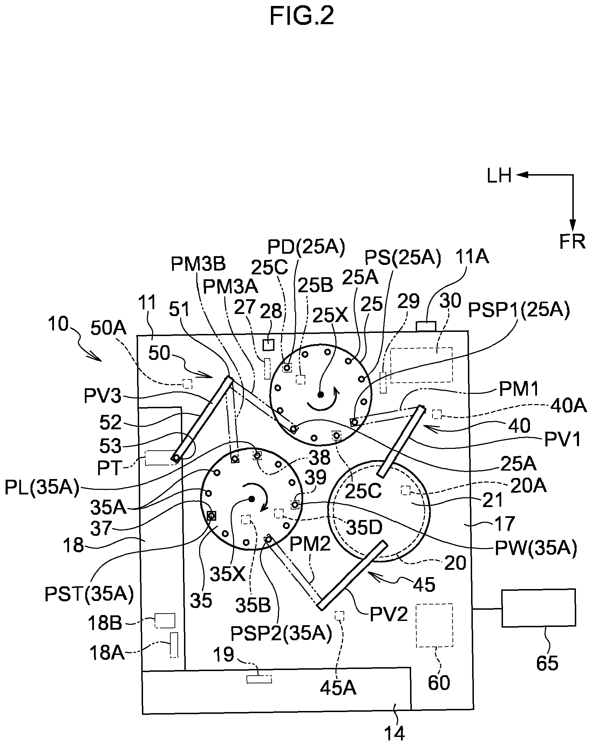

As shown in Fig. 1, an operation panel 15 is provided at a front end portion of the top plate 12. The operation panel 15 includes a start button 16A, an input unit 16B for inputting various instruction contents, and a device stop button 16C. As shown in Fig. 2, a main switch 11A is provided on the back surface of the analysis device 10. In a case in which the main switch 11A is at an off-position, power of the power supply is not supplied to the analysis device 10. On the other hand, in a case in which the main switch 11A is at an on-position, the power of the power supply is supplied to the analysis device 10.

As shown in Figs. 1 and 2, the housing 11 includes a support plate 17, located below the top plate 12 and parallel to the top plate 12, in the internal space. As shown in Fig. 2, the support plate 17 is provided with a rack transfer mechanism 18, which extends in the front-rear direction in plan view, in a left portion. The rack transfer mechanism 18 moves, to a specimen collection position PT shown in Fig. 2, the specimen racks 70 installed in the rack tray 14A one by one in the order of installation. For example, the rack transfer mechanism 18 may include a rail (not shown) extending in the front-rear direction, a moving body 18B capable of reciprocating in the front-rear direction along the rail by a driving force of a first electric motor 18A, and an arm portion (not shown) supported by the moving body 18B and capable of gripping the specimen racks 70 one by one. However, a configuration different from this configuration may be adopted as the configuration of the rack transfer mechanism 18. In a case in which the specimen rack 70 located at the specimen collection position PT is returned to the rack tray 14A, the rack transfer mechanism 18 moves another specimen rack 70 installed next to the specimen rack 70 returned to the rack tray 14A to the specimen collection position PT.

As shown in Fig. 2, the support plate 17 is provided with a reader 19 capable of reading the rack barcode 72 and the specimen barcode 76. In a case in which the rack tray 14A is set in the rack accommodating portion 14, the rack transfer mechanism 18 transfers the specimen racks 70 to a reading position of the reader 19, the reader 19 reads the rack barcode 72 provided in each of the specimen racks 70 and the specimen barcode 76 of each of the specimen containers 75, and the read information is transmitted from the reader 19 to a control unit 60 to be described later.

As shown in Fig. 1, the top plate 12 is provided with a first reagent probe 40 and a second reagent probe 45. As shown in Fig. 2, the support plate 17 is provided with a reagent cooler 20, a first disk (first measurement unit) (first support unit) 25, a second disk (second measurement unit) (second support unit) 35, a stirrer 37, a photometric unit 38 (second measurement execution unit), a cleaning unit 39, and a specimen probe (specimen dispensing mechanism) 50.

The reagent cooler 20 having a columnar shape is supported by the support plate 17 so as to be located immediately below the first opening 13A of the top plate 12. A top opening of the reagent cooler 20 is opened and closed by a lid member 21. That is, the reagent cooler 20 (lid member 21) is exposed above the support plate 17 through the first opening 13A. Furthermore, the reagent cooler 20 has a cooling function for maintaining the quality of various reagents stored in the reagent cooler 20. These reagents include a reagent for coagulation measurement (a first measurement reagent, not shown) and a reagent for colorimetric measurement (a second measurement reagent, not shown). Here, a stirrer 20A (see Fig. 2), capable of stirring the reagents by, for example, a magnetic force is provided below the reagent cooler 20. The reagent cooler 20 includes a rotatable reagent disk therein, and the reagent disk has a structure in which a plurality of reagent containers can be installed at equal angular intervals.

The first disk 25 having a circular shape is rotatably supported by the support plate 17 so as to be located immediately below the second opening 13B of the top plate 12. As shown in Fig. 2, a plurality of container-supporting portions 25A are provided on a top of the first disk 25 at equal angular intervals in the circumferential direction (not shown in Fig. 1). In each of the container-supporting portions 25A, a cuvette (first reaction container) 26 (see Fig. 1) of a disposable type for coagulation measurement is placed. Each of the cuvettes 26 has an open top. The first disk 25 is rotatable in the counterclockwise direction in Fig. 2 about a central axis 25X by the driving force of a second electric motor 25B. The first disk 25 repeats an operation of stopping after rotation by a predetermined angle and rotating again by the predetermined angle after a lapse of a predetermined stop time. That is, the first disk 25 intermittently rotates. For example, in a case in which a total number of the container-supporting portions 25A is N1, the predetermined angle is a value (angle) obtained by dividing 360° by N1.

Each of the container-supporting portions 25A is provided with a photometric unit (first measurement unit) (first measurement execution unit) 25C (see Fig. 2) including an LED light source and a photodetector that detects scattered light of a solution in the cuvette, and a thermostatic unit (first temperature adjustment unit) 25D that maintains the cuvette in a predetermined temperature range (part of the thermostatic units 25D is shown in Fig. 1).

As shown in Fig. 2, the support plate 17 is provided with a disposal unit 28 located near the first disk 25. The disposal unit 28 is an opening at one end of a passage provided inside the analysis device 10, and the other end of the passage faces a collection box (not shown) detachably provided in the housing 11. The cuvette 26 transferred to a disposal position PD (see Fig. 2), which is a position on the first disk 25, is introduced into the disposal unit 28 by a first conveyance mechanism (first measurement unit) (first movement unit) (conveyance mechanism) 27 (see Fig. 2), and the introduced cuvette 26 is collected by the collection box. The position of the disposal position PD is fixed. That is, a relative position of the disposal position PD with respect to the housing 11 is invariable. The same applies to a photometric position PL, a stirring position PST, a container supply position PS, a reagent supply position PSP1, a reagent supply position PSP2, and a cleaning position PW to be described later. Every time the first disk 25 rotates by the predetermined angle, one container-supporting portion 25A coincides with the disposal position PD, another container-supporting portion 25A coincides with the container supply position PS, and still another container-supporting portion 25A coincides with the reagent supply position PSP1.

As shown in Fig. 2, the support plate 17 is provided with a container supply unit 30 located near the first disk 25. The container supply unit 30 accommodates unused cuvettes 26. Every time the first disk 25 stops rotating, one cuvette 26 in the container supply unit 30 is conveyed to the container supply position PS (container-supporting portion 25A), which is a position on the first disk 25, by a second conveyance mechanism (conveyance mechanism) 29.

The second disk 35 having a circular shape is rotatably supported by the support plate 17 so as to be located immediately below the third opening 13C of the top plate 12. A plurality of container-supporting portions 35A are provided on a top of the second disk 35 at equal angular intervals in the circumferential direction (see Fig. 2, not shown in Fig. 1). In each of the container-supporting portions 35A, a light-transmitting cell (second reaction container) 36 is placed (see Fig. 1). Each of the cells 36 has an open top. The second disk 35 is rotatable in the clockwise direction in Fig. 2 about a central axis 35X by a driving force of a third electric motor 35B. The second disk 35 repeats rotation and stop such that the cells 36 sequentially move through predetermined positions in accordance with measurement steps. The second disk 35 includes a thermostatic unit (second temperature adjustment unit) 35C for maintaining the cells 36 in a predetermined temperature range therein (see Fig. 1).

The stirrer 37, the photometric unit 38, and the cleaning unit 39 are provided near the second disk 35. The stirrer 37 is disposed immediately above the stirring position PST which is a position on the second disk 35. The photometric unit 38 is disposed at the photometric position PL which is a position facing a side surface of the second disk 35. The photometric unit 38 includes a white light source (halogen lamp) and a photodetector that detects light (absorbance) transmitted through the cell 36. The cleaning unit 39 is disposed at the cleaning position PW which is a position on the second disk 35. The second disk 35 repeats rotation and stop such that one container-supporting portion 35A coincides with the stirring position PST, another container-supporting portion 35A coincides with the photometric position PL, and still another container-supporting portion 35A coincides with the cleaning position PW.

Next, the first reagent probe 40 will be described. The first reagent probe 40 includes a rotation shaft 41 rotatably supported by the top plate 12, a first arm 42 extending in the horizontal direction from an upper end of the rotation shaft 41, and a first pipette 43 extending downward from a distal end portion of the first arm 42. The rotation shaft 41 can reciprocate in the vertical direction with respect to the top plate 12 and can rotate about its own axis by a driving force of a fourth electric motor 40A. The first pipette 43 is connected to a suction and discharge unit (not shown). The first reagent probe 40 is rotatable between a first suction position PV1 indicated by a solid line in Fig. 2 and a first discharge position PM1 indicated by a virtual line in Fig. 2. The first reagent probe 40 is movable in the vertical direction between a first standby position where a lower end of the first pipette 43 is located above the top plate 12 and a first lowered position where a lower end of a second pipette 48 approaches the support plate 17 through the opening 13.

Next, the second reagent probe 45 will be described. The second reagent probe 45 includes a rotation shaft 46 rotatably supported by the top plate 12, a second arm 47 extending in the horizontal direction from an upper end of the rotation shaft 46, and the second pipette 48 extending downward from a distal end portion of the second arm 47. The rotation shaft 46 can reciprocate in the vertical direction with respect to the top plate 12 and can rotate about its own axis by a driving force of a fifth electric motor 45A. The second pipette 48 is connected to a suction and discharge unit. The second reagent probe 45 is rotatable between a second suction position PV2 indicated by a solid line in Fig. 2 and a second discharge position PM2 indicated by a virtual line in Fig. 2. The second reagent probe 45 is movable in the vertical direction between a second standby position where the lower end of the second pipette 48 is located above the top plate 12 and a second lowered position where the lower end of the second pipette 48 approaches the support plate 17 through the opening 13.

Next, the specimen probe 50 will be described. The specimen probe 50 is provided in a space between the top plate 12 and the support plate 17. The specimen probe 50 includes a rotation shaft 51 that is rotatably supported by the support plate 17 and extends in the vertical direction, a third arm 52 extending in the horizontal direction from an upper end of the rotation shaft 51, and a third pipette 53 extending downward from a distal end portion of the third arm 52. The rotation shaft 51 can reciprocate in the vertical direction with respect to the support plate 17 and can rotate about its own axis by a driving force of a sixth electric motor 50A. The third pipette 53 is connected to a suction and discharge unit. The specimen probe 50 is rotatable between a third suction position PV3 indicated by a solid line in Fig. 2 and third discharge positions PM3A and PM3B indicated by virtual lines in Fig. 2. The specimen probe 50 is movable in the vertical direction between a third standby position where a lower end of the third pipette 53 is greatly separated upward from the support plate 17 and a third lowered position closer to the support plate 17 than the third standby position.

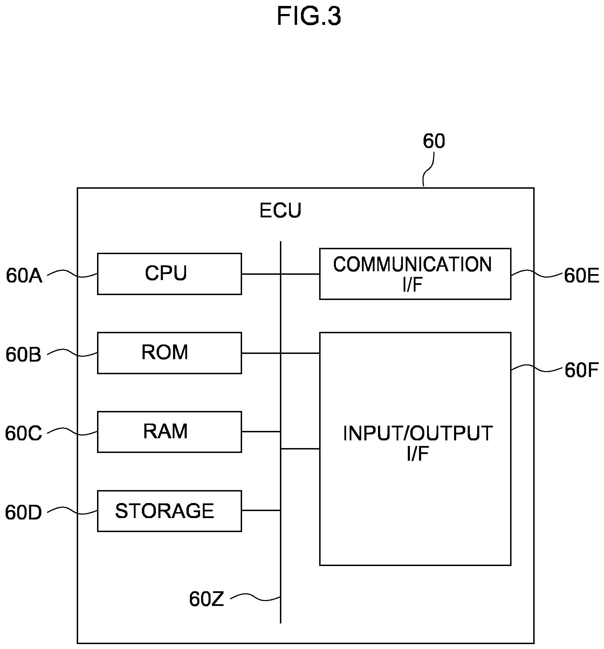

As shown in Fig. 2, the housing 11 is provided with the control unit 60. The control unit 60 is electrically connected to the operation panel 15, the reader 19, the stirrer 20A, the photometric unit 25C, the thermostatic unit 25D, the first conveyance mechanism 27, the second conveyance mechanism 29, the thermostatic unit 35C, the water supply unit 35D (described later), the stirrer 37, the photometric unit 38, the cleaning unit 39, the first electric motor 18A, the second electric motor 25B, the third electric motor 35B, the fourth electric motor 40A, the fifth electric motor 45A, the sixth electric motor 50A, and the respective suction and discharge units. As illustrated in Fig. 3, the control unit 60 includes a central processing unit (CPU) (processor) 60A, a read only memory (ROM) 60B, a random access memory (RAM) 60C, a storage (recording unit) 60D, a communication inter face (I/F) 60E, and an input/output I/F 60F. The CPU 60A, the ROM 60B, the RAM 60C, the storage 60D, the communication I/F 60E, and the input/output I/F 60F are connected to communicate with each other via a bus 60Z.

The CPU 60Ais a central processing unit, and executes various programs and controls each unit. That is, the CPU 60A reads the programs from the ROM 60B or the storage 60D, and executes the programs using the RAM 60C as a work area. The CPU 60A performs control of each of the components and various types of arithmetic processing according to the programs recorded in the ROM 60B or the storage 60D.

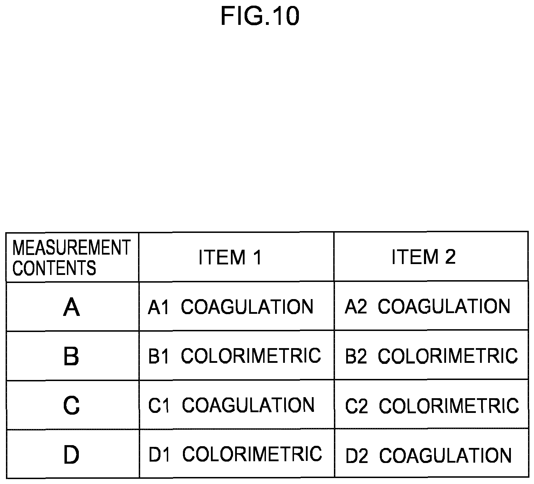

The ROM 60B stores various programs and various types of data. The RAM 60C temporarily stores programs or data as a work area. The storage 60D includes a storage device such as a hard disk drive (HDD) or a solid state drive (SSD), and stores various programs and various types of data. For example, the storage 60D records a measurement order list in which information of the specimen barcode 76 is associated with a measurement item for each specimen. The measurement order list is created based on, for example, measurement request information associated with information of the specimen barcode 76 read by the reader 19 and recorded in a host computer (not shown) or measurement request information input by the input unit 16B, and tables of Figs. 10 to 12 to be described later. Measurement by the analysis device 10 is executed using the measurement order list. The measurement order list includes information regarding the measurement order of measurement items of each specimen determined according to a predetermined rule. That is, in the measurement order list, information regarding the order of measurement of each of a plurality of measurement items in a case in which the measurement items are performed on one specimen is recorded. The communication I/F 60E is an interface for the control unit 60 to communicate with the host computer and the like.

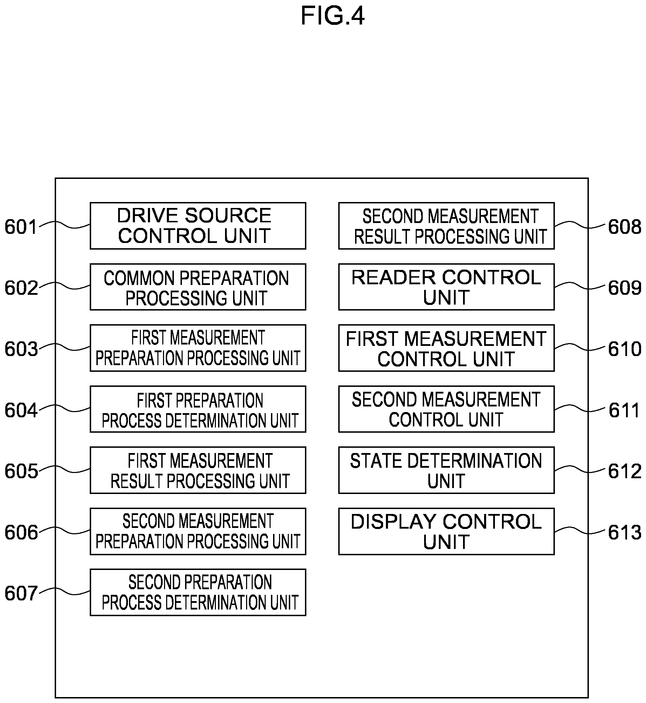

Fig. 4 is a block diagram showing an example of functional components of the control unit 60. The control unit 60 includes, as the functional components, a drive source control unit 601, a common preparation processing unit 602, a first measurement preparation processing unit 603, a first preparation process determination unit 604, a first measurement result processing unit 605, a second measurement preparation processing unit 606, a second preparation process determination unit 607, a second measurement result processing unit 608, a reader control unit 609, a first measurement control unit 610, a second measurement control unit 611, a state determination unit 612, and a display control unit 613. These functions are implemented as the CPU 60A reads and executes the programs stored in the ROM 60B. Note that "first" and "second" at the head of a name of each unit represents "coagulation" and "colorimetric", respectively.

The drive source control unit 601 controls the first electric motor 18A, the second electric motor 25B, the third electric motor 35B, the fourth electric motor 40A, the fifth electric motor 45A, the sixth electric motor 50A, the first conveyance mechanism 27, the second conveyance mechanism 29, and the respective suction and discharge units.

The common preparation processing unit 602 executes a common preparation process before execution of coagulation measurement and colorimetric measurement by controlling various device components provided in the analysis device 10. The common preparation process includes the following process A1 to process A4.
Process A1: Process of confirming supply of system water
Process A2: Process of confirming operations of first reagent probe 40, second reagent probe 45, and specimen probe 50
Process A3: Reagent disk rotation operation and home position confirmation operation
Process A4: Process of confirming remaining amount of reagent installed in reagent cooler 20

The first measurement preparation processing unit 603 executes a coagulation measurement preparation process after completion of the common preparation process by controlling various device components provided in the analysis device 10. The coagulation measurement preparation process includes the following processes B1 to B7.
Process B 1: Process of confirming amount of scattered light.
Process B2: Process of stirring reagent using stirrer 20A provided below reagent cooler 20 with respect to reagent that needs to be stirred before measurement in order to uniformize reagent in reagent container
Process B3: Attaching operation process of cuvette 26 to container-supporting portion 25A using second conveyance mechanism 29 and detaching operation process of cuvette 26 from container-supporting portion 25A using first conveyance mechanism 27
Process B4: Process of confirming operation of supplying cuvette 26 from inside of container supply unit 30.
Process B5: Process of confirming operation of disposing of cuvette 26
Process B6: Rotation operation confirmation process and home operation process of first disk 25
Process B7: Thermostatic control process by thermostatic unit 25D provided on first disk 25

The first preparation process determination unit 604 determines whether a first preparation process, which is a preparation process including the common preparation process and the coagulation measurement preparation process, is completed. Note that the first preparation process is also referred to as a warming up process in the following description.

In a case in which coagulation measurement is completed, the coagulation measurement result processing unit 605 processes a measurement result of the coagulation measurement, records a processing result in the storage 60D, and outputs the processing result to a display 65 to be described later.

The second measurement preparation processing unit 606 can start a colorimetric measurement preparation process simultaneously with the coagulation measurement preparation process after completion of the common preparation process by controlling various device components provided in the analysis device 10. In order to cope with a case in which it is known in advance that there is no colorimetric measurement for a specimen scheduled to be measured (for example, in a case in which measurement items of all the specimen containers 75 (specimens) are only coagulation items), it may be possible to set whether or not the second measurement preparation processing unit 606 executes the colorimetric measurement preparation process. The colorimetric measurement preparation process includes the following processes C1 to C7. In the following description, the colorimetric measurement preparation process is also referred to as a start up process.
Process C1: Process of confirming light source of photometric unit 38 (including confirmation of accumulated use time and confirmation of amount of light)
Process C2: Process of confirming remaining amount of detergent supplied to cleaning unit 39
Process C3: Rotation operation confirmation process and home operation process of second disk 35
Process C4: Process of cleaning all cells 36 using cleaning unit 39
Process C5: Water blank measurement process of dispensing water into cells 36 using water supply unit 35D (system water supply unit) (see Fig. 2) and measuring light absorbance of water in cells using photometric unit 38
Process C6: Process of determining contamination states of cells 36 using water blank measurement results
Process C7: Temperature control confirmation process (thermostatic control process) of thermostatic unit 35 C provided in second disk 35

The second preparation process determination unit 607 determines whether a second preparation process, which is a preparation process including the common preparation process and the colorimetric measurement preparation process, is completed. Note that a time required for the colorimetric measurement preparation process is longer than a time required for the coagulation measurement preparation process. Therefore, the time at which the preparation process ends is later in the second preparation process than in the first preparation process. For example, a time required for the second preparation process is about 5 minutes, and a time required for the first preparation process is about 2 minutes.

In a case in which colorimetric measurement is completed, the colorimetric measurement result processing unit 608 processes a measurement result of the colorimetric measurement, records a processing result in the storage 60D, and outputs the processing result to the display 65.

The reader control unit 609 controls the reader 19.

The coagulation measurement control unit 610 controls the photometric unit 25C and the thermostatic unit 25D provided in the container-supporting portion 25A when coagulation measurement is executed.

The colorimetric measurement control unit 611 controls the stirrer 37, the photometric unit 38, and the cleaning unit 39 provided in the second disk 35 when colorimetric measurement is executed.

The state determination unit 612 recognizes various states of the analysis device 10. These states include, for example, an on/off state of the main switch 11A, a sleep state in which the analysis device 10 is automatically activated at a set time, a warming-up (WARMING UP) state, a pre-ready (PRE READY) state, a ready (READY) state, and a start-up (START UP) state. The warming-up state, the pre-ready state, and the ready state will be described later.

The display control unit 613 controls the display 65 to be described later.

As shown in Fig. 2, the display 65 is connected to the analysis device 10. The display 65 can display various types of information. Examples of the information include information on a measurement request content requested to the analysis device 10, information indicating a measurement result of coagulation measurement output by the coagulation measurement result processing unit 605, and information indicating a measurement result of colorimetric measurement output by the colorimetric measurement result processing unit 608.

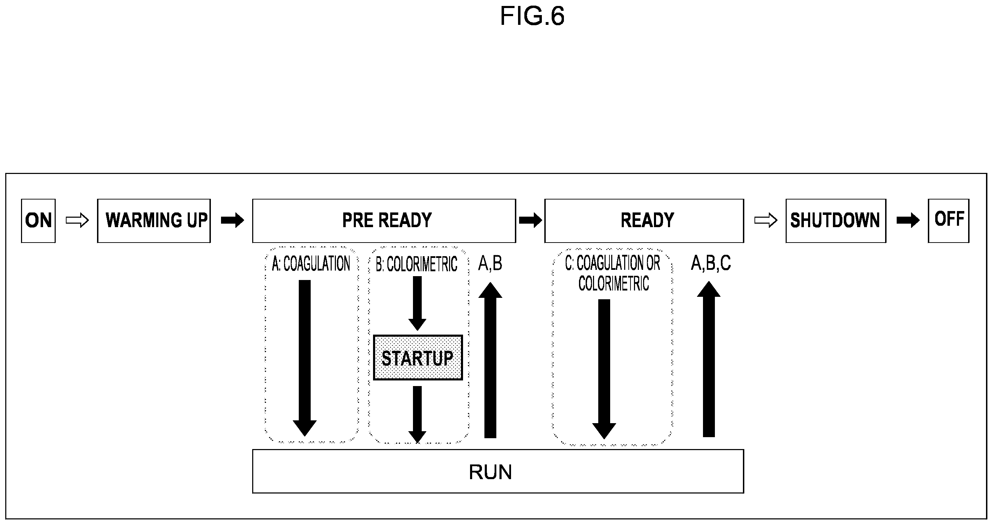

Next, various states of the analysis device 10 will be described with reference to Fig. 6.

WARMING UP: A state in which an operation of initializing each unit of analysis device 10 and a measurement preparation process (such as the common preparation process described above) are being executed. The analysis device 10 transitions from [ON] to [WARMING UP] by turning the main switch 11A on or automatically turning the main switch 11A on at a set time from the sleep state (not shown).

PRE READY: A state in which the start button 16Afor starting measurement is allowed to be pressed although the measurement preparation process of colorimetric measurement is not completed. Measurement of a coagulation item (A of the drawing) is performed according to the measurement request content. However, [START UP] is required in measurement of a colorimetric item (B of the drawing). When the common preparation process described above is completed in the WARMING UP process, a transition from [WARMING UP] to [PRE READY] is automatically made.

READY: A state in which the start button 16A is allowed to be pressed. [START UP] is not required in the measurement of the colorimetric item, and the colorimetric item (C in the drawing) is also measured according to the measurement request content similarly to the coagulation item.

When the measurement preparation process (start-up process) of colorimetric measurement is completed, a transition from [PRE READY] to [READY] is automatically made.

START UP: A state during the measurement preparation process of colorimetric measurement. A transition from [PRE READY] to [START UP] is made by input of the start button 16A or automated start based on detection of installation of the specimen rack 70 in the rack tray 14A.

RUN: A state in which measurement is being executed (from acquisition of measurement request information to output of a measurement result). A transition from [PRE READY] to [RUN] or from [READY] to [RUN] is made by input of the start button 16A or automated start based on detection of installation of the specimen rack 70 in the rack tray 14A. Alternatively, when the measurement preparation process (start-up process) of colorimetric measurement is completed, a transition from [START UP] to [RUN] is automatically made. After measurement results of all types of measurement for which measurement requests have been received are output, a transition from [RUN] to [PRE READY] or from [RUN] to [READY] is automatically made.

SHUTDOWN: A state during post-processing for stopping the analysis device 10. A transition from [READY] to [SHUT DOWN] is made by input of the device stop button 16C In addition, a transition from [SHUT DOWN] to [OFF] is made by turning off the power supply of the analysis device 10 or by bringing the analysis device 10 into the sleep state (not shown) through input of a sleep button or the like.

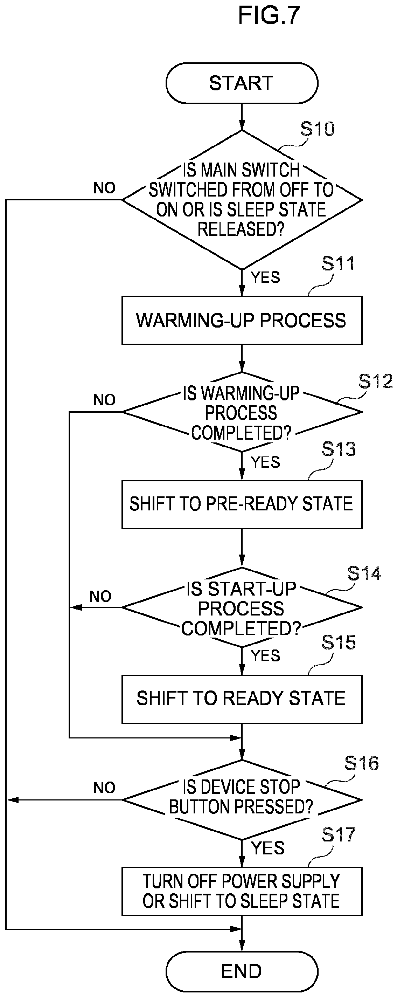

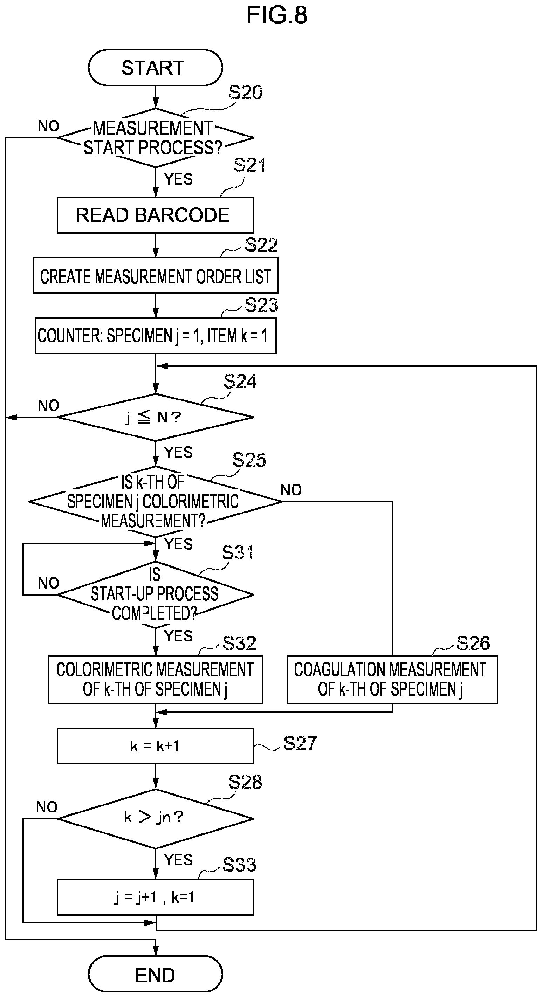

Next, operations of the analysis device 10 will be described with reference to flowcharts of Figs. 7 and 8. First, the flowchart of Fig. 7 will be described. The CPU 60A repeatedly executes processing of the flowchart of Fig. 7 every time a predetermined time elapses.

First, in step S10, the CPU 60A determines whether the main switch 11A is switched from the off-state to the on-state or whether the analysis device 10 is switched from the sleep state to a normal state.

In a case in which Yes is determined in step S10, the CPU 60A proceeds to step S11 and starts the warming-up (WARMING UP) process. That is, the CPU 60A starts a coagulation measurement preparation process in a case in which the common preparation process described above is completed. The CPU 60A starts a colorimetric measurement preparation process (start-up process) in a case in which the common preparation process is completed.

Next, in step S12, the CPU 60A determines whether the warming-up process is completed.

In a case in which Yes is determined in step S12, the CPU 60A proceeds to step S13 and shifts the analysis device 10 to the pre-ready (PRE READY) state. That is, the CPU 60A brings the analysis device 10 into a state in which coagulation measurement is possible.

Next, in step S14, the CPU 60A determines whether the start-up process (colorimetric preparation process) is completed.

In a case in which Yes is determined in step S14, the CPU 60A proceeds to step S15 and causes the analysis device 10 to transition to the ready (READY) state. That is, the CPU 60A brings the analysis device 10 into a state in which colorimetric measurement is possible.

Next, in step S16, the CPU 60A determines whether the device stop button 16C is pressed.

In a case in which Yes is determined in step S16, the CPU 60A proceeds to step S 17, and executes a shutdown process or switches the analysis device 10 to the sleep state.

In a case in which the process in step S17 is completed or in a case in which No is determined in step S10 or 16, the CPU 60A temporarily ends the process of the flowchart of Fig. 7.

Next, the flowchart of Fig. 8 will be described. In a case in which the analysis device 10 is in the pre-ready state or the ready state, the CPU 60A repeatedly executes processing of the flowchart of Fig. 8 every time a predetermined time elapses.

First, the CPU 60A determines whether a measurement start process is executed in step S20. That is, the CPU 60A determines whether the start button 16A is pressed or whether a start process using a touch panel (not shown) provided in the analysis device 10 is executed. For example, in a case in which the start button 16A is pressed, the CPU 60A determines Yes in step S20. Note that the CPU 60A may determine Yes in step S20 in a case in which the specimen rack 70 is set in the rack tray 14A.

It is assumed that the specimen rack 70 shown in Fig. 9 is to be set in the rack tray 14A. Each of the specimen racks 70 can support five specimen containers 75. However, it is assumed that two specimen containers 75 are supported by each of the specimen racks 70 in the following description. That is, N = 2 as described later. It is assumed that the number of measurement items executed for a specimen in each of the specimen containers 75 is "2". That is, k = 2 as described later.

In a case in which Yes is determined in step S20, the CPU 60A proceeds to step S21, the rack transfer mechanism 18 transfers the specimen rack 70-1 to the reading position of the reader 19, and causes the reader 19 to read the rack barcode 72 and each of the specimen barcodes 76. The CPU 60A inquires the host computer about a measurement request content for each specimen container based on the read information of each of the specimen barcodes 76, and receives the measurement request content from the host computer.

Next, the CPU 60A further controls the first electric motor 18A. As a result, the rack transfer mechanism 18 transfers the specimen rack 70-1 to the specimen collection position PT in Fig. 2.

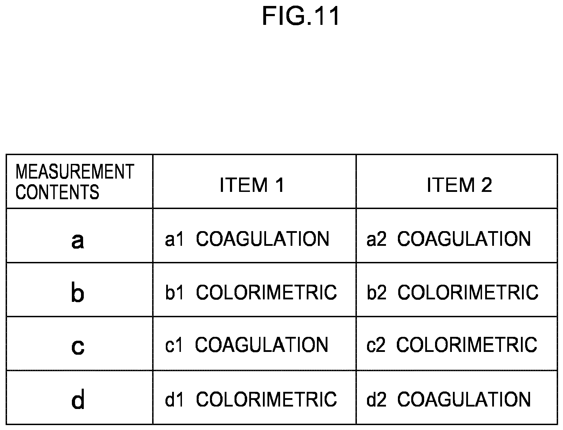

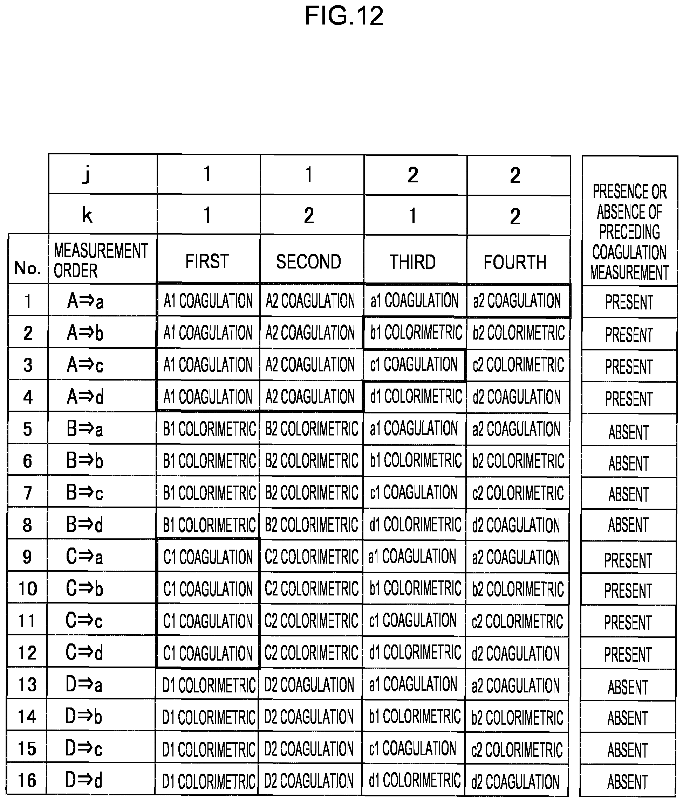

The CPU 60A having finished the process in step S21 proceeds to step S22, and creates a measurement order list based on the measurement request content of each specimen received from the host computer and the tables of Figs. 10 to 12. Note that data representing the tables of Figs. 10 to 12 is recorded in, for example, the storage 60D. Fig. 10 shows combinations of measurement items performed on the specimen in the specimen container 75-1. That is, the number of the combinations in this case is four (A, B, C, and D). In Fig. 10, "Item 1" is an item to be measured first, and "Item 2" is an item to be measured second. Fig. 11 shows combinations of measurement items performed on the specimen in the specimen container 75-2. That is, the number of the combinations in this case is four (a, b, c, and d). In Fig. 11, "Item 1" is an item to be measured first, and "Item 2" is an item to be measured second. Fig. 12 shows combinations of measurement items performed on the two specimen containers 75-1 and 75-2 supported by each of the specimen racks 70. That is, the number of combinations in this case is sixteen. Here, j represents the measurement order of each of the specimen containers 75 (specimens). In this case, a specimen with j = 1 is the specimen in the specimen container 75-1 of each of the specimen racks 70, and a specimen with j = 2 is the specimen in the specimen container 75-2 of each of the specimen racks 70. In this case, the total number (N) of the specimens is "2 (pieces)". Here, k represents a measurement item to be measured k-th in each of the specimen containers 75 (k is hereinafter also referred to as the number of measurement items). A measurement item to be measured first for a specimen is represented by k = 1, and a measurement item to be measured second for the specimen is represented by k = 2. For example, in the case of Nos. 9 to 12 in Fig. 12, the measurement item of k = 1 of the specimen of j = 1 is the coagulation item, and the measurement item of k = 2 of the specimen with j = 1 is the colorimetric item.

Next, in step S23, the CPU 60A sets count values of j and k to "1" which is an initial value.

Next, in step S24, the CPU 60A determines whether j ≤ N. Since N = 2 and j = 1, the CPU 60A determines Yes in step S24 and proceeds to step S25.

Next, in step S25, the CPU 60A determines whether the k-th measurement item of the specimen container 75 of j = 1 is colorimetric measurement. For example, in a case in which the measurement item of k = 1 with j = 1 in the measurement order list is the coagulation item (Nos. 1 to 4 and 9 to 12 in Fig. 12), the CPU 60A determines No in step S25 and proceeds to step S26 to execute the first coagulation measurement of the specimen of j = 1. In a case in which the measurement item of k = 1 with j = 1 in the measurement order list is the colorimetric item (Nos. 5 to 8 and 13 to 16 in Fig. 12), the CPU 60A determines Yes in step S25, proceeds to step S31, and determines whether the start-up process is completed.

In step S26, the CPU 60A executes a series of steps of the coagulation measurement (specimen dispensing, reagent dispensing, reaction process measurement, coagulation time calculation, concentration and activity calculation as necessary, and measurement result output as necessary). The CPU 60A executes at least specimen dispensing among the series of steps of the coagulation measurement. First, the specimen probe 50 is moved to the third discharge position PM3A after sucking the specimen from the specimen container 75 installed in the specimen rack 70-1 at the specimen collection position PT by the third pipette 53, and discharges the specimen to the cuvette 26 supported by the container-supporting portion 25A. The cuvette 26 containing the discharged specimen moves to the reagent supply position PSP1. Next, the first reagent probe 40 is moved to the first discharge position PM1 after sucking the first measurement reagent stored in the reagent cooler 20 by the first pipette 43, and the first measurement reagent is discharged to the cuvette 26 containing the specimen and located at the reagent supply position PSP1. A sample solution including the specimen and the first measurement reagent discharged into the cuvette 26 is temperature-controlled by the thermostatic unit 25D.

The CPU 60A controls the photometric unit 25C provided in the container-supporting portion 25A. As a result, the light source irradiates the sample solution in the cuvette 26 with light, and the photodetector receives scattered light generated by scattering the light incident on the sample solution. The CPU 60A records a measurement result received from the photometric unit 25C in the storage 60D in association with ID information of the specimen container 75, and displays the measurement result on the display 65.

In step S31, the CPU 60A determines whether the start-up process is completed and proceeds to step S32 to execute the colorimetric measurement in a case in which Yes is determined. The CPU 60A executes at least specimen dispensing in a series of steps of the colorimetric measurement.

In step S32, the CPU 60A executes the series of steps (specimen dispensing, reagent dispensing, reaction process measurement, concentration and activity calculation, and measurement result output) of the colorimetric measurement. First, the specimen probe 50 is moved to the third discharge position PM3B after sucking the specimen from the specimen container 75 installed in the specimen rack 70-1 at the specimen collection position PT by the third pipette 53, and discharges the specimen to the cell 36 supported by the container-supporting portion 35A. The cell 36 containing the discharged specimen moves to the reagent supply position PSP2. Next, the second reagent probe 45 moves to the second discharge position PM2 after sucking the second measurement reagent stored in the reagent cooler 20 by the second pipette 48, and discharges the second measurement reagent to the cell 36 containing the specimen and located at the reagent supply position PSP2. The sample solution including the specimen and the second measurement reagent discharged to the cell is temperature-controlled by the thermostatic unit 35C.

In a case in which the cell 36 containing the sample solution moves to the stirring position PST in Fig. 2, the stirrer 37 stirs the sample solution in the cell 36. In a case in which the cell 36 further moves to the photometric position PL in Fig. 2, the light source of the photometric unit 38 irradiates the sample solution in the cell 36 with light, and the photodetector detects transmitted light transmitted through the sample solution. Then, the CPU 60A records a measurement result received from the photometric unit 28 in the storage 60D in association with ID information of the specimen container 75, and displays the measurement result on the display 65.

In a case in which the process in step S26 or step S32 is completed, the CPU 60A proceeds to step S27 and adds "1" to a count number of k. In this case, the CPU 60A proceeds to processing of the second measurement item (k = 2) of the specimen with j = 1.

Next, in step S28, the CPU 60A determines whether k > jn. Here, jn is a maximum value of the number of measurement items (k) of the specimen containers 75 to be measured, and jn = 2 in the present embodiment.

In this case, since k = 2, the CPU 60A determines No in step S28 and returns to step S24. Then, the CPU 60A proceeds to processing of the measurement item of the specimen of j = 1 and k = 2, that is, the measurement item to be measured second in the specimen container 75-1 installed in the specimen rack 70-1. At this time, in a case in which the measurement item of the specimen of j = 1 and k = 2 is the coagulation item, the CPU 60A performs the same processes as those in steps S24, S25, and S26 described above on the specimen. In a case in which the measurement item of the specimen of j = 1 and k = 2 is the colorimetric item, the CPU 60Aperforms the same processes as those in steps S24, S25, S31, and S32 described above on the specimen.

In a case in which the process in step S28 is performed thereafter, the CPU 60A determines Yes in step S28 since j = 1 and k = 3 and proceeds to step S33. In step S33, the CPU 60A counts up j (j = j+1) and returns k to the initial value (k = 1) in order to proceed to the processing of the specimen (j = 2) to be measured next. In this case, j = 2 and k = 1. Thereafter, the CPU 60A returns to step S24, and proceeds to processing of a measurement item of the specimen of j = 2 and k = 1, that is, a measurement item to be measured first in the specimen container 75-2 installed in the specimen rack 70-1. At this time, in a case in which the measurement item of the specimen of j = 2 and k = 1 is the coagulation item, the CPU 60A performs the same processes as those in steps S24, S25, S26, and S27 described above on the specimen. In a case in which the measurement item of the specimen of j = 2 and k = 1 is the colorimetric item, the CPU 60A performs the same processes as those in steps S24, S25, S31, S32, and S27 described above on the specimen.

In a case in which the process in step S24 is performed on the specimen of j = 3 and k = 1, the CPU 60A determines No, the rack transfer mechanism 18 returns the specimen rack 70-1 located at the specimen collection position PT to the rack tray 14A, and the CPU 60A temporarily ends the processing of the flowchart of Fig. 8. Next, the CPU 60A performs the same processing as that of the flowchart of Fig. 8 described above on the specimen rack 70-2 to be measured next. The same applies to the specimen racks 70-3 to 70-10 in Fig. 1.

As described above, the analysis device 10 of the present embodiment performs coagulation measurement on a specimen in the cuvette 26 after the first preparation process is completed. After completion of the second preparation process, the analysis device 10 performs colorimetric measurement on the specimen in the cell 36. The time required for the second preparation process is longer than the time required for the first preparation process. In a case in which the first preparation process is completed, the analysis device 10 performs the coagulation measurement regardless of whether the second preparation process is completed. That is, the analysis device 10 can perform the coagulation measurement in a case in which the second preparation process is not completed and the first preparation process is completed. Therefore, the analysis device 10 can execute the coagulation measurement at an earlier timing as compared with a case in which the coagulation measurement is performed after completion of the first preparation process and the second preparation process. Therefore, the analysis device 10 can efficiently execute the coagulation measurement and the colorimetric measurement on the specimen (for example, Nos. 1 to 4 and 9 to 12 in Fig. 12).

In a case in which a measurement item of the specimen container 75 is only the coagulation measurement (for example, Nos. 1 to 4 in Fig. 12), the analysis device 10 executes the coagulation measurement regardless of whether the second preparation process is completed in response to completion of the first preparation process. Also in a case in which measurement items include the coagulation measurement and only the colorimetric measurement executed after the coagulation measurement (for example, Nos. 3 and 9 to 12 in Fig. 12), the analysis device 10 executes the coagulation measurement regardless of whether the second preparation process is completed in response to completion of the first preparation process. Therefore, the analysis device 10 can efficiently execute the coagulation measurement and the colorimetric measurement not only in the case in which the measurement item of one specimen container 75 is only the coagulation measurement but also in the case in which the measurement items include the coagulation measurement and only the colorimetric measurement executed after the coagulation measurement.

In the analysis device 10, the first disk 25 for performing the coagulation measurement and the second disk 35 for performing the colorimetric measurement are separated from each other. Therefore, the analysis device 10 can perform the coagulation measurement preparation process and the colorimetric measurement preparation process independently of each other. Therefore, it is easy to configure the analysis device 10 such that the coagulation measurement can be performed without waiting for completion of the second preparation process.

Here, an analysis device (not shown) of a comparative example including a single disk that performs coagulation measurement and colorimetric measurement is assumed. In the analysis device of the comparative example, a coagulation measurement preparation process and a colorimetric measurement preparation process are executed on the single disk. Therefore, in the analysis device of the comparative example, it is not possible to execute the coagulation measurement on the disk until the colorimetric measurement preparation process is completed. That is, it is not possible for the analysis device of the comparative example to efficiently execute the coagulation measurement and the colorimetric measurement on the specimen.

In the analysis device 10, the cuvette 26 on which the coagulation measurement is executed is the container of the disposable type. That is, the water blank measurement process for determining the contamination state and the cleaning process, which are required in the cell 36, are unnecessary for the cuvette 26. Therefore, the time required for the coagulation measurement preparation process can be made shorter than the time required for the colorimetric measurement preparation process.

Although the embodiment of the disclosure has been described above, the disclosure is not limited to this embodiment.

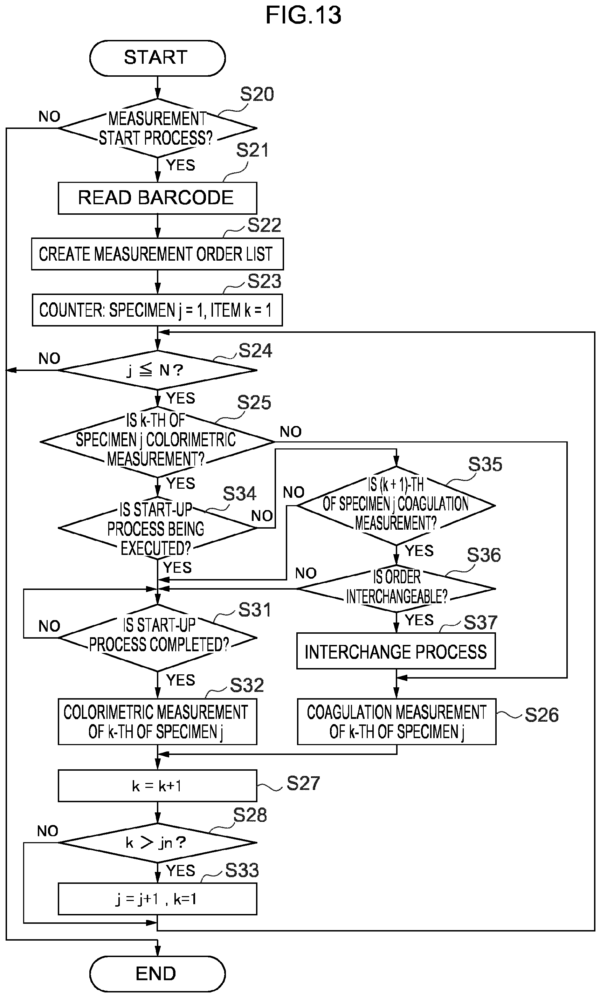

For example, the analysis device 10 may be controlled using a flowchart of a modification shown in Fig. 13. The flowchart of Fig. 13 is obtained by adding a plurality of steps to the flowchart of Fig. 8. In the flowchart of Fig. 13, in a case in which the CPU 60A determines Yes in step S25, the CPU 60A proceeds to step S34 and determines whether the analysis device 10 is performing the start-up process. In a case in which No is determined in step S34, the CPU 60A proceeds to step S35 and determines whether the (k + 1)-th measurement item of a specimen as a specimen target is the coagulation measurement. That is, in a case in which the common preparation process is completed but the start-up process (colorimetric preparation process) is not started, the CPU 60A proceeds to step S35. For example, in a case in which the first measurement is performed on the specimen in the specimen container 75 whose measurement content in Fig. 10 is "D", the CPU 60A determines Yes in step S35. Next, in step S36, the CPU 60A determines whether the order of the first (k-th) and second ((k + 1)-th) measurement items may be interchanged based on a measurement order list. The measurement order list of the present modification includes information regarding whether the order of measurement items may be interchanged. In a case in which Yes is determined in step S36, the CPU 60A proceeds to step S37 and interchanges the order of the first (k-th) and second ((k + 1)-th) measurement items. Next, the CPU 60A proceeds to step S26, and performs the coagulation measurement scheduled to be performed second ((k+ 1)-th) as the first (k-th) measurement item. Therefore, according to the modification, the analysis device 10 can more efficiently execute the coagulation measurement and the colorimetric measurement on the specimen as compared with the embodiment.

The processing of the flowchart of Fig. 8 or 13 may be executed on the specimen rack 70 supporting one specimen container 75 or three or more specimen containers 75.

For example, processing contents of the common preparation process, the coagulation measurement preparation process, and the colorimetric measurement preparation process may be different from the processing contents.

The rack tray 14A may be configured to enable placement of one specimen rack 70 or a plurality of (different from ten) specimen racks 70. In addition, the specimen rack 70 may include one support groove 71 or a plurality of (different from five) support grooves 71.

The analysis device 10 may perform measurement on three or more measurement request items for plasma (a specimen) put in one specimen container 75. For example, the analysis device 10 may perform the coagulation measurement once and perform the colorimetric measurement twice on the plasma put in one specimen container 75.

In a case in which measurement is performed on the specimen in the specimen container 75 whose measurement content in Fig. 10 is "C", the analysis device 10 may start the colorimetric measurement without waiting for completion of the coagulation measurement. For example, before the coagulation cuvette 26 located at the disposal position PD is moved to the disposal unit 28 and the coagulation cuvette 26 in the container supply unit 30 is moved to the container supply position PS, the second pipette 48 may dispense the second measurement reagent for the colorimetric measurement into the colorimetric cuvettes 36. Similarly, in a case in which measurement is performed on the specimen in the specimen container 75 whose measurement content in Fig. 10 is "D", the analysis device 10 may start the coagulation measurement without waiting for completion of colorimetric measurement. For example, before the display 65 displays an analysis result of the colorimetric measurement, the first pipette 43 may dispense the first measurement reagent for the coagulation measurement into the coagulation cuvettes 26.

The analysis device 10 may be configured to execute the coagulation measurement and measurement (specific measurement) different from the colorimetric measurement and the coagulation measurement on the specimen.

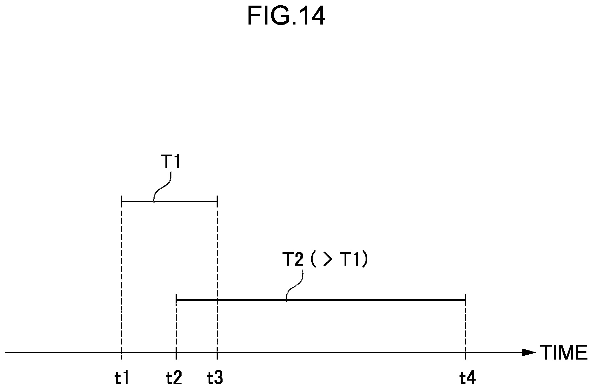

Furthermore, the disclosure may be implemented in an aspect of a modification shown in Fig. 14. In Fig. 14, T1 represents a time required for the coagulation measurement preparation process. In this example, the coagulation measurement preparation process starts at time t1 and ends at time t3. In Fig. 14, T2 is a time required for a preparation process (hereinafter, also referred to as specific preparation process) other than the common preparation process in a case in which the specific measurement is performed, and T2 > T1. That is, the specification preparation process corresponds to the colorimetric measurement preparation process in a case in which the colorimetric measurement is executed. In this example, the specification preparation process is started at time t2 after the time t1 and ends at time t4 after the time t3. The analysis device 10 of the modification can also execute the coagulation measurement at an earlier timing as compared with a case in which the coagulation measurement is performed under a condition of completion of the specific preparation process.

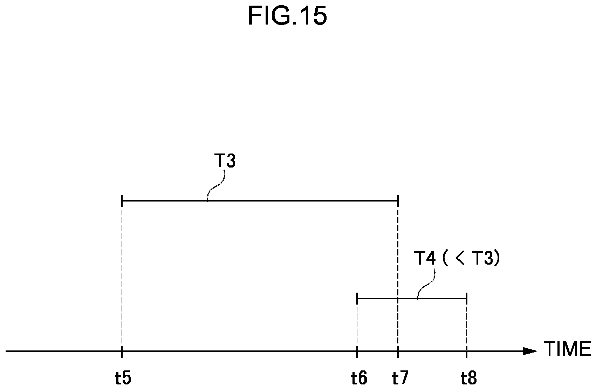

Furthermore, the disclosure may be implemented in an aspect of a modification shown in Fig. 15. In Fig. 15, T3 represents a time required for the coagulation measurement preparation process. In this example, the coagulation measurement preparation process starts at time t5 and ends at time t7. In Fig. 15, T4 is a time required for the specific preparation process, and T4 < T3. In this example, the specification preparation process is started at time t6 after the time t5 and ends at time t8 after the time t7. The analysis device 10 of the modification can also execute the coagulation measurement at an earlier timing as compared with a case in which the coagulation measurement is performed under a condition of completion of the specific preparation process.

The number of specimen containers subjected to the measurement processes by the analysis device 10 may be one.

The disclosure of Japanese Patent Application No. 2022-056531 filed on March 30, 2022 is incorporated herein by reference in its entirety. All cited documents, patent applications, and technical standards mentioned in the present specification are incorporated by reference in the present specification to the same extent as if the individual cited document, patent application, or technical standard was specifically and individually indicated to be incorporated by reference.

### Description of Reference Numerals

10 Automated analysis device (analysis device)
25 First disk (first measurement unit) (first support unit)
25A Container-supporting portion
25C Photometric unit (first measurement unit) (first measurement execution unit)
25D Thermostatic unit (first temperature adjustment unit)
26 Cuvette (first reaction container)
27 First conveyance mechanism (first measurement unit) (first movement unit) (conveyance mechanism)
29 Second conveyance mechanism (conveyance mechanism)
30 Container supply unit
35 Second disk (second measurement unit) (second support unit)
35A Container-supporting portion
35C Thermostatic unit (second temperature adjustment unit)
35D Water supply unit (system water supply unit)
36 Cell (second reaction container)
38 Photometric unit (second measurement unit) (second measurement execution unit) (contamination detection unit)
39 Cleaning unit (second measurement unit)
50 Specimen probe (specimen dispensing mechanism)
60D Storage (recording unit)
75(75-1, 75-2, 75-3, 75-4, 75-5) Specimen container

## Claims

1. An automated analysis device comprising:
a recording unit that records one or more measurement items performed on a specimen placed in at least one specimen container and records a measurement order of each of the measurement items;
a specimen dispensing mechanism that dispenses the specimen selected as a measurement target based on the measurement order into at least one of a first reaction container or a second reaction container;
a first measurement unit that performs coagulation measurement, which is measurement of a coagulation item, on the specimen dispensed into the first reaction container after completion of a first preparation process determined in advance; and
a second measurement unit that performs specific measurement, which is a measurement item that is different from the measurement of the coagulation item, on the specimen dispensed into the second reaction container after completion of a second preparation process determined in advance,
wherein the first measurement unit is capable of executing the measurement of the coagulation item regardless of whether the second preparation process is completed, in response to the completion of the first preparation process.

2. The automated analysis device according to claim 1, wherein a time required for the second preparation process is longer than a time required for the first preparation process.

3. The automated analysis device according to claim 1 or 2, wherein the first measurement unit and the second measurement unit are separated from each other.

4. The automated analysis device according to claim 3, wherein:
the first measurement unit includes:
a first support unit that supports a plurality of first reaction containers into which the specimen dispensed by the specimen dispensing mechanism is placed,
a first measurement execution unit that performs the coagulation measurement on the specimen in the first reaction containers, and
a first movement unit that moves the first reaction containers on which the coagulation measurement has been executed from the first support unit to an outside of the first support unit without cleaning;
the second measurement unit includes:
a second support unit that supports a plurality of second reaction containers into which the specimen dispensed by the specimen dispensing mechanism is placed,
a second measurement execution unit that performs the specific measurement on the specimen in the second reaction containers, and
a cleaning unit that cleans the second reaction containers on which the specific measurement has been executed; and
the second preparation process includes a cleaning process which is performed on all of the second reaction containers by the cleaning unit.

5. The automated analysis device according to any one of claims 1 to 4, wherein the first measurement unit performs the coagulation measurement regardless of whether the second preparation process is completed, in response to the completion of the first preparation process in a case in which the measurement item performed on the specimen in the specimen container is only the coagulation measurement or includes the coagulation measurement and only the specific measurement executed after the coagulation measurement.

6. The automated analysis device according to any one of claims 1 to 5, wherein the first preparation process includes a process of stirring a reagent used for the coagulation measurement.

7. The automated analysis device according to any one of claims 1 to 6, further comprising:
a first support unit that is a part of the first measurement unit and supports a plurality of first reaction containers into which the specimen dispensed by the specimen dispensing mechanism is placed;
a container supply unit that stores the plurality of first reaction containers; and
a conveyance mechanism that is capable of conveying the first reaction containers accommodated in the container supply unit to the first support unit and conveying the first reaction containers from the first support unit to a predetermined position,
wherein the first preparation process includes a process of conveying the first reaction containers to the first support unit using the conveyance mechanism and a process of conveying the first reaction containers from the first support unit using the conveyance mechanism.

8. The automated analysis device according to any one of claims 1 to 7, further comprising:
a first support unit that is a part of the first measurement unit and supports a plurality of first reaction containers into which the specimen dispensed by the specimen dispensing mechanism is placed; and
a first temperature adjustment unit that performs thermostatic control of a temperature of the first support unit at a constant temperature,
wherein the first preparation process includes a thermostatic control process of the first support unit by the first temperature adjustment unit.

9. The automated analysis device according to any one of claims 1 to 8, further comprising:
a second support unit that is a part of the second measurement unit and supports a plurality of second reaction containers into which the specimen dispensed by the specimen dispensing mechanism is placed; and
a cleaning unit that cleans the second reaction containers supported by the second support unit,
wherein the second preparation process includes a process of cleaning all of the second reaction containers supported by the second support unit, the process being executed by the cleaning unit.

10. The automated analysis device according to any one of claims 1 to 9, further comprising:
a second support unit that is a part of the second measurement unit and supports a plurality of second reaction containers into which the specimen dispensed by the specimen dispensing mechanism is placed; and
a system water supply unit that supplies system water to the second reaction containers,
wherein the second preparation process includes a system water measurement process by the second measurement unit.

11. The automated analysis device according to any one of claims 1 to 10, further comprising:
a second support unit that is a part of the second measurement unit and supports a plurality of second reaction containers into which the specimen dispensed by the specimen dispensing mechanism is placed; and
a contamination detection unit that detects a contamination state of the second reaction containers,
wherein the second preparation process includes a contamination detection process of the second reaction containers by the contamination detection unit.

12. The automated analysis device according to any one of claims 1 to 11, further comprising:
a second support unit that is a part of the second measurement unit and supports a plurality of second reaction containers into which the specimen dispensed by the specimen dispensing mechanism is placed; and
a second temperature adjustment unit that performs thermostatic control of a temperature of the second support unit,
wherein the second preparation process includes a thermostatic control process of the second support unit by the second temperature adjustment unit.
